(19) Europäisches Patentamt
European Patent Office
Office européen des brevets

(11) Publication number: **0 171 265**
**B1**

(12) **EUROPEAN PATENT SPECIFICATION**

(45) Date of publication of patent specification: **18.07.90**

(51) Int. Cl.$^5$: **C 07 C 17/12,** B 01 J 29/28

(21) Application number: **85305498.9**

(22) Date of filing: **01.08.85**

# (54) Process for preparing halogenated benzene derivatives.

(30) Priority: **01.08.84 JP 160274/84**

(43) Date of publication of application:
**12.02.86 Bulletin 86/07**

(45) Publication of the grant of the patent:
**18.07.90 Bulletin 90/29**

(84) Designated Contracting States:
**DE FR GB NL**

(56) References cited:
**EP-A-0 112 722**

**Kirk-Othmer, Encyclopedia of chemical technology, vol. 15 (1981), 657-658**

(73) Proprietor: **Tosoh Corporation**
**4560, Oaza Tonda**
**Shinnanyo-shi Yamaguchi-ken (JP)**

(72) Inventor: **Sekizawa, Kazuhiko**
**972 Oaza Tonda**
**Shinnanyo-shi Yamaguchi (JP)**
Inventor: **Hironaka, Toshio**
**2068, Oaza Heta**
**Tokuyama-shi Yamaguchi (JP)**
Inventor: **Tsutsumi, Yukihiro**
**1723, Oaza Shimogami**
**Tokuyama-shi Yamaguchi (JP)**

(74) Representative: **Lawrence, Peter Robin Broughton et al**
**GILL JENNINGS & EVERY 53-64 Chancery Lane**
**London WC2A 1HN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 171 265 B1

**Description**

The present invention relates to a process for preparing a halogenated benzene derivative by halogenating benzene and/or a benzene derivative in the presence of a zeolite as a catalyst. More particularly, it relates to a process for preparing a p-substituted halogenated benzene derivative by halogenating benzene and/or a benzene derivative by using a zeolite-containing catalyst formed by incorporating a binder with a zeolite.

Halogenated benzenes are industrially important compounds, and especially dichlorobenzene (hereinafter referred to as "DCB") is used as a starting compound for the production of valuable compounds such as pigments. DCB includes three isomers, that is, ortho-, meta- and para-isomers, and p-dichlorobenzene (hereinafter referred to as "PDCB") is used not only as the starting compound for the production of various compounds but also directly as an insecticide or deodorizer.

Ordinarily, DCB is prepared according to the liquid-phase chlorination process in which benzene or monochlorobenzene (hereinafter referred to as "MCB") is chlorinated in the presence of a Lewis acid, such as ferric chloride, by introducing chlorine into the reaction mixture.

It is known that since the chlorination reaction of MCB shows a strong orientation, in the liquid-phase chlorination process using a Lewis acid, o-dichlorobenzene (hereinafter referred to as "ODCB") and PDCB are selectively formed. In this reaction, ODCB is formed at a ratio of 30 to 40% and PDCB is formed at a ratio of 60 to 70%. However, since the demand for PDCB is larger than that for ODCB, it is desired to develop a process in which PDCB is selectively prepared while controlling the formation of ODCB.

As the process for halogenating an aromatic compound in the presence of a zeolite catalyst, there can be mentioned, for example, a liquid-phase halogenation process in which a halogenated benzene is brominated in the liquid phase, and a process for selectively preparing a para-isomer by using various ion-exchanged faujasites, that is, X type and Y type zeolites, as the catalyst [see Journal of Catalysis, *60*, pages 110—120 (published in 1979)].

As the gas-phase halogenation process, there is known a process for chlorinating benzene in the gas phase by using a zeolite such as ZSM-5, mordenite, L type zeolite or Y type zeolite as a catalyst (Tetrahedron Letters, *21*, pages 3809—3812, 1980).

As pointed out above, it has been clarified that zeolites shown an effective catalytic activity for halogenation of aromatic compounds.

Ordinarily, zeolites are prepared in the form of a crystalline fine powder. Accordingly, if such a crystalline fine zeolite is directly used as a practical catalyst or industrial catalyst, pressure loss is caused or the difficult operation of separating the catalyst becomes necessary to prevent incorporation of the catalyst into a product. Accordingly, where the catalyst component is in the powdery form, the catalyst component is molded into an appropriate shape or size, for example, into a spherical or columnar pellet or a granule, according to the reaction state (liquid phase or gas phase), the manner of use of the catalyst (fixed bed, suspended bed or fluidized bed), the reaction manner (batchwise reaction or continuous reaction) and the shape of the reaction vessel. On the other hand, the performance, stability, and durability, i.e., the life, of the catalyst under application conditions are very important. Especially, in a molded catalyst, mechanical strength characteristics such as a high degree of hardness, high powdering resistance, and high abrasion resistance are required, and a good moldability including a high molding efficiency is required for molding the catalyst. Ordinarily, a binder is used for satisfying these requirements.

Since a zeolite powder is non-caking, a synthetic inorganic oxide such as silica or alumina or a clay such as natural montmorillonite, purified montmorillonite, kaolin or acid clay is often used for molding the zeolite powder. Especially in view of the mechanical strength characteristics of a molded catalyst and the moldability, an alumina type oxide is frequently used. Furthermore, most of the clays used contain an aluminum compound such as aluminum oxide.

As seen from the foregoing description, it is eagerly desired in the art to develop a zeolite catalyst excellent as an industrial catalyst and to prepare PDCB selectively while controlling the formation of ODCB.

The present inventors carried out research into practical zeolite catalysts for the halogenation of aromatic compounds, and as a result, found that if an aluminum compound other than the aluminum constituent of the zeolite is present in a molded zeolite catalyst in an amount exceeding a certain level, the selectivity to a para-substituted compound is drastically reduced. Since a zeolite is ordinarily prepared from silicon and aluminum compounds as the main starting materials, it is possible that the unreacted aluminum compound is often left as an impurity in the formed zeolite. Accordingly, the aluminum compound may be present in the molded catalyst irrespective of the kind of binder used and this aluminum compound reduces the catalytic performance. Surprisingly, it was found that the selectivity to PDCB remains high when the halogenation of benzene and/or a benzene derivative is effected by using a zeolite-containing catalyst containing a specified amount of an aluminum compound other than the zeolite component in the catalyst.

In accordance with the present invention, there is provided a process for the preparation of halogenated benzene derivatives, which comprises halogenating benzene or a benzene derivative in the presence of a zeolite catalyst. The zeolite catalyst is characterized as being a zeolite-containing catalyst which contains an aluminum compound other than the zeolite component in the catalyst in an amount of 0.01 to 30% by weight as $Al_2O_3$.

The main component of the zeolite-containing catalyst used in the present invention is a zeolite. As the zeolite, there can be mentioned faujasite type zeolites such as X type and Y type zeolites, offretite-erionite type zeolites, L type zeolite, mordenite, ferrierite and ZSM-5 type zeolites. Of these, L type zeolite and offretite-erionite type zeolites are preferable.

The L type zeolite has a powder X-ray diffraction pattern (Kα doublet of copper) shown in Table 1, by which the L type zeolite is distinguishable from other zeolites.

TABLE 1

| Diffraction angle 2θ (°) (±0.2°) | Lattice spacing d (Å) (±0.1 Å) | Relative intensity I/Ic |
|---|---|---|
| 5.6 | 15.8 | 100 |
| 11.2 | 7.89 | 5—40 |
| 11.8 | 7.49 | 10—100 |
| 14.8 | 5.98 | 10—100 |
| 15.3 | 5.79 | 10—40 |
| 19.4 | 4.57 | 30—80 |
| 20.2 | 4.39 | 10—70 |
| 20.5 | 4.33 | 10—70 |
| 22.7 | 3.91 | 30—120 |
| 23.4 | 3.80 | 5—40 |
| 24.3 | 3.66 | 10—70 |
| 25.6 | 3.48 | 20—80 |
| 27.2 | 3.28 | 10—60 |
| 28.0 | 3.18 | 30—120 |
| 29.1 | 3.07 | 20—80 |
| 29.8 | 3.00 | 5—40 |
| 30.8 | 2.91 | 20—80 |
| 33.8 | 2.65 | 10—70 |
| 34.2 | 2.62 | 5—50 |

The L type zeolite is a known zeolite and can be synthesized according to a known process. A typical composition of the L type zeolite is expressed by the following oxide molar ratio formula:

$$xM_{2/n}O \cdot Al_2O_3 \cdot ySiO_2 \cdot zH_2O$$

wherein M is a cation, n is a valency of the cation M, x is a number of 0.7 to 1.3, y is a number of 4 to 8, and z is a number varying depending upon the extent of hydration of the zeolite and is ordinarily from 0 to 9. A typical example of the L type zeolite has a composition expressed by the following oxide molar ratio formula:

$$M_2O \cdot Al_2O_3 \cdot 6SiO_2 \cdot 5H_2O$$

wherein M is sodium and/or potassium.

The as-prepared L type zeolite can be used as the catalyst, but all or a part of the metal cation (M) contained in the as-prepared L type zeolite may be ion-exchanged with another metal cation and/or proton before it is used as the catalyst. The ion exchange treatment may be accomplished, for example, by suspending the zeolite in an aqueous solution containing the intended metal cation and stirring the suspension at 20°C to 100°C for 1 to 50 hours.

As the offretite-erionite type zeolite, there can be mentioned offretite, tetramethylammonium-offretite (hereinafter referred to as "TMA-offretite"), erionite, and zeolites in which both offretite and erionite structures are present, such as a novel zeolite called "zeolite OE" (EP—0106643), zeolite T, and ZSM-34 zeolite. The zeolite of this type has a characteristic powder X-ray diffraction pattern, by which the zeolite is distinguishable from other zeolites. The X-ray diffraction pattern by the Kα doublet of copper of the offretite-erionite type zeolite is shown in Table 2. Although some of the zeolites of this type are naturally produced, zeolites of this type can be synthesized and such synthesis processes are known.

TABLE 2

| Diffraction angle 2θ (°) (±0.2°) | Lattice spacing d (Å) (±0.1 Å) | Relative intensity I/Io |
|---|---|---|
| 7.7 | 11.5 | 100 |
| 9.6 | 9.2 | 0—10 |
| 11.7 | 7.6 | 10—40 |
| 13.4 | 6.6 | 40—90 |
| 14.1 | 6.3 | 10—40 |
| 15.4 | 5.7 | 10—40 |
| 16.6 | 5.3 | 0—10 |
| 19.4 | 4.6 | 10—60 |
| 20.5 | 4.3 | 40—80 |
| 21.3 | 4.2 | 0—10 |
| 23.3 | 3.8 | 20—70 |
| 23.7 | 3.8 | 100—160 |
| 24.8 | 3.6 | 50—120 |
| 26.1 | 3.4 | 5—30 |
| 26.9 | 3.3 | 10—40 |
| 27.6 | 3.2 | 0—10 |
| 28.1 | 3.2 | 10—40 |
| 28.3 | 3.2 | 20—60 |
| 30.5 | 2.9 | 5—20 |
| 31.2 | 2.9 | 50—120 |
| 31.4 | 2.8 | 50—130 |
| 31.9 | 2.8 | 0—10 |
| 33.4 | 2.5 | 10—30 |
| 35.9 | 2.5 | 0—30 |
| 36.2 | 2.5 | 5—30 |

The offretite-erionite type zeolite has a composition represented by the following oxide molar ratio formula:

$$xM_{2/n}O \cdot Al_2O_3 \cdot ySiO_2 \cdot zH_2O$$

wherein M is a cation, n is a valency of the cation M, x is a number of 0.7 to 1.3, y is a number of 5 to 10, and z is a number varying depending upon the extent of hydration of the zeolite and is ordinarily from 0 to 10.

A synthetic zeolite having a low impurity content and a high crytallinity is preferred as the offretite-erionite type zeolite used as the catalyst.

The as-synthesized offretite-erionite type zeolite ordinarily contains a metal cation M such as a sodium ion or a potassium ion, but it is known that this metal cation can be easily exchanged with another metal cation by an ion exchange treatment. It is preferred that the exchangeable cation of the offretite-erionite type zeolite used in the present invention be exchanged with a metal cation selected from potassium, rubidium, cesium, calcium, strontium, and barium. It is sufficient if one of the foregoing metal cations is present in the zeolite, but two or more of these metal cations may be simultaneously present in the zeolite. It is preferred that the ion exchange ratio of the metal cation, that is, the ratio of exchange of the exchangeable cation with the metal cation, be at least 70%, more preferably at least 90%. If at least two metal cations are present in the zeolite, it is preferred that the sum of the ion exchange ratios be at least 70%, more preferably at least 90%. If the ion exchange ratio is lower than 70%, a satisfactory selectivity to the para-substituted compound cannot be obtained. The metal cation can be easily introduced into the zeolite by a known ion exchange treatment. If the synethesized offretite-erionite type zeolite is one having an organic cation such as TMA, to increase the ion exchange ratio with the metal cation, it is preferred that the organic cation be removed in advance. This removal can be accomplished by performing a preliminary calcining treatment in an air current at a temperature of from 400°C to 700°C for 1 to 10 hours.

The X-type zeolite has a composition represented by the following oxide molar ratio formula:

$$xM_{2/n}O \cdot Al_2O_3 \cdot ySiO_2 \cdot zH_2O$$

wherein M is a cation, n is a valency of the cation M, x is a number of 0.7 to 1.3, y is a number of 2 to 3, and z is a number varying depending upon the extent of hydration of the zeolite and is ordinarily from 0 to 11.

The Y-type zeolite has a composition represented by the following oxide molar ratio formula:

$$xM_{2/n}O \cdot Al_2O_3 \cdot ySiO_2 \cdot zH_2O$$

wherein M is a cation, n is a valency of the cation M, x is a number of 0.7 to 1.3, y is a number of 3 to 6, and z is a number varying depending upon the extent of hydration of the zeolite and is ordinarily from 0 to 20.

Mordenite has a composition represented by the following oxide molar ratio formula:

$$xM_{2/n}O \cdot A_2O_3 \cdot ySiO_2 \cdot zH_2O$$

wherein M is a cation, n is a valency of the cation M, x is a number of 0.7 to 1.3, y is a number of 10 to 20, and z is a number varying depending upon the extent of hydration of the zeolite and is ordinarily from 0 to 11.

Ferrierite has a composition represented by the following oxide molar ratio formula:

$$xM_{2/n}O \cdot Al_2O_3 \cdot ySiO_2 \cdot zH_2O$$

wherein M is a cation, n is a valency of the cation M, x is a number of 0.7 to 1.3, y is a number of 10 to 100, and z is a number varying depending upon the extent of hydration of the zeolite and is ordinarily from 0 to 10.

The ZSM-5 zeolite has a composition represented by the following oxide molar ratio formula:

$$xM_{2/n}O \cdot Al_2O_3 \cdot ySiO_2 \cdot zH_2O$$

wherein M is a cation, n is a valency of the cation M, x is a number of 0.7 to 1.3, y is a number of 10 to 1000, and z is a number varying depending upon the extent of hyration of the zeolite and is ordinarily from 0 to 40.

It is preferable that the zeolite used as the catalyst has a high purity. If the above-mentioned method is adopted, a zeolite having a high purity is ordinarily obtained. The purity of the zeolite can be known from the peak intensity of the powder X-ray diffraction pattern.

The zeolite is ordinarily used in a molded form as a catalyst in the process of the present invention. The shape and dimension of the molded catalyst are appropriately determined according to the mode of the reaction, and ordinarily molding methods such as extrusion molding, compression molding, and spray-drying granulation can be appropriately adopted. Since the zeolite is a non-caking powder, as pointed out above, a binder is used for molding. Binders customarily used in this field, for example, synthetic inorganic oxides such as alumina and silica-alumina, and natural and refined clays such as kaolin, acid clay, montmorillonite, and sepiolite, may be used in the present invention. The kind of the binder is not particularly critical. In view of the mechanical strength and reaction activity of the molded catalyst, the binder is used in an amount of 10 to 70% by weight, based on the molded catalyst. In the process of the present invention, the content of an aluminum compound in the zeolite-containing catalyst, other than the alumina component constituting the zeolite, is 0.01 to 30% by weight, expressed as $Al_2O_3$. If the amount of the $Al_2O_3$ other than the zeolite is too large and exceeds the range specified in the present invention, the selectivity to a para-substituted compound in the halogenation reaction is drastically reduced. Even if an aluminum compound-free binder, such as silica, magnesia, titania or silica-magnesia, is used as the binder for the formation of the catalyst, an aluminum compound is sometimes included in the molded catalyst according to the conditions used for the preparation of the zeolite. Accordingly, in this case also, the above requirement of the aluminum compound content should be satisfied.

The catalyst used in the present invention may be prepared according to known molding methods, and the molding method is not particularly critical. It is generally preferred that the as-molded catalyst be dried and calcined at a temperature of from 300°C to 700°C before it is used for the reaction.

In the present invention, the term "benzene derivative" means a compound in which hydrogen of benzene is substituted with a substituent such as a halogen atom or an alkyl group, for example, halogenobenzene or alkylbenzene. As specific examples, there can be mentioned monofluorobenzene, MCB, monobromobenzene (hereinafter referred to as "MBB"), monoiodobenzene, toluene, and ethylbenzene. An elementary halogen may be used as the halogenating agent. For example, chlorine, bromine, and iodine can be mentioned. The halogenation reaction of benzene and/or a benzene derivative according to the present invention may be carried out either in the liquid phase or in the gas phase.

When the process of the present invention is carried out in the liquid phase, the reaction apparatus or reaction condition is not particularly critical, in so far as benzene and/or a benzene derivative is placed in contact in the liquid state with the catalyst. For example, either a batchwise reaction vessel or a continuous reaction vessel may be used. The reaction may be carried out in the presence of a solvent not participating in the halogenation reaction, such as carbon tetrachloride. If a solvent is used, it is preferred that the concentration of benzene and/or a benzene derivative be 5 to 100%, especially 10 to 100%. If the concentration is lower than 5%, the chance of contact of the starting compound with the catalyst is undesirably reduced.

Where a halogenating agent is continuously supplied, an inert gas such as nitrogen, helium or carbon dioxide may be fed together with the halogenating agent. It is preferred that the concentration of the halogenating agent be 5 to 100%, more preferably 10 to 100%.

When a batchwise or semi-batchwise reaction vessel is used, usually the catalyst is used in the state

wherein it is suspended in the solution, and it is preferred that the amount of the catalyst per unit of liquid volume be 0.001 to 1 kg/l, more preferably 0.005 to 0.1 kg/l. If the amount of the catalyst is smaller than 0.001 kg/l, the load on the catalyst is too large and a satisfactory activity cannot be obtained. When the halogenating agent is continuously supplied, it is preferred that the feed rate of the halogenating agent (the feed rate per unit of liquid volume) be 1 to 1000 l/hr · l, more preferably 5 to 500 l/hr · l. If the feed rate is lower than 1 l/hr · l, a sufficient rate of reaction cannot be obtained. If the feed rate is higher than 1000 l/hr · l, a diffusion rate-controlled state is brought about and a substantial increase of the effect cannot be attained by an increase of the feed rate.

The reaction temperature or reaction pressure is not particularly critical, in so far as the benzene and/or a benzene derivative is in the liquid phase. When the reaction temperature is higher than the boiling point of benzene and/or a benzene derivative, the halogenation reaction may be conducted in the liquid phase by elevating the pressure.

It is preferred that the reaction temperature be from 0°C to 400°C, especially from 20°C to 300°C. If the reaction temperature is lower than 0°C, a sufficient rate of reaction cannot be obtained. At a temperature higher than 400°C, the selectivity to a para-substituted halogenated benzene derivative is reduced.

Where the process of the present invention is carried out in the gas phase, the reaction vessel is not particularly critical and an ordinary fixed bed can be used. Note, the process of the present invention still may be carried out even if a fluidized bed or moving bed is used.

In carrying out the gas phase reaction, the molar ratio of the halogenating agent to the benzene derivative is 0.01 to 5.0 mole/mole, preferably 0.1 to 2.0 mole/mole. If the molar ratio of the halogenating agent to the benzene derivative is lower than 0.01 mole/mole, the yield of a para-substituted halogenated benzene derivative is undesirably low. If this molar ratio is higher than 5.0 mole/mole, the amount of a polyhalogenated benzene formed as a by-product is increased.

The gas phase reaction may be performed by using only benzene and/or a benzene derivative and the halogenating agent, or a diluent gas may be incorporated. Any of diluent gases inactive to the reaction of the present invention may be used. For example, nitrogen, helium, and hydrogen can be mentioned. The concentration of the starting compound is 1 to 100%, preferably 1 to 50%.

The starting material mixture may be supplied so that the contact time W/F [g-cat · hr/mole; W is the weight (g) of the catalyst and F is the total feed rate (mole/hr) of benzene and/or a benzene derivative and the halogenating agent] is 5 to 500 g-cat · hr/mole, preferably 10 to 100 g-cat · hr/mole. If the contact time is smaller than 5 g-cat · hr/mole, a satisfactory conversion of the benzene derivative cannot be obtained. If the contact time exceeds 500 g-cat · hr/mole, the amount of a polyhalogenated benzene formed as a by-product is increased.

The reaction temperature should be 100°C to 400°C, preferably 150°C to 300°C. If the reaction temperature is lowere than 100°C, a sufficient conversion cannot be obtained. At a temperature higher than 400°C, the selectivity to a para-substituted halogenated benzene is reduced. The reaction pressure is not particularly critical, and any pressure condition may be adopted, in so far as the reaction is advanced in the gas phase.

The present invention will now be described in detail with reference to the following examples that by no means limit the scope of the invention.

In the examples, the conversion and selectivity are values calculated according to the following equations.

$$\text{Conversion (\%)} = \frac{A-B}{A} \times 100$$

A: amount (millimoles) of charged or supplied benzene derivative
B: amount (millimoles) of unreacted benzene derivative

$$\text{Selectivity} = \frac{C}{D} \times 100$$

C: amount (millimoles) of intended product formed
D: sum (millimoles) of all products formed.

Example 1

To an overflow type reaction vessel equipped with a stirrer, a sulfuric acid-acidified aqueous solution of aluminum sulfate ($Al_2O_3$=4.44 w/v%, $H_2SO_4$=25.69 w/v%) and an aqueous solution of sodium silicate ($Na_2O$=6.56 w/v%, $SiO_2$=20 w/v%, $Al_2O_3$=0.22 w/v%) were simultaneously and continuously supplied at feed rates of 0.25 l/hr and 0.75 l/hr, respectively. Reaction was carried out with stirring. The residence time of the reaction slurry was adjusted to 30 minutes and the reaction was conducted at a pH value of 6.2 and a temperature of 32°C. The slurry product overflowing from the reaction vessel was subjected to solid-liquid separation, and the recovered solid was washed with water to obtain an amorphous compound of the following composition.

| | |
|---|---|
| $Na_2O$: | 5.2% by weight (dry base) |
| $Al_2O_3$: | 7.13% by weight (dry base) |
| $SiO_2$: | 87.7% by weight (dry base) |
| $H_2O$: | 59.7% by weight (wet base) |

To 176 g of an aqueous solution of potassium hydroxide having a concentration of 16.4% by weight was added 142.2 g of the above amorphous compound. The mixture was stirred to form a slurry reaction mixture. This reaction mixture was charged in an autoclave and maintained at 170°C under an autogenerous pressure for 24 hours to effect crystallization. After completion of the reaction, the formed solid was separated, washed with water and dried at 110°C. From the results of chemical analysis, it was found that the product had the following composition expressed by the molar ratio of oxides (dry base):

$$0.99\ K_2O \cdot 0.01Na_2O \cdot Al_2O_3 \cdot 6.0SiO_2$$

The powder X-ray diffraction pattern of the sample, shown in Table 3, was substantially in accord with the pattern shown in Table 1, and it was confirmed that the product was an L type zeolite. Note, the powder X-ray diffraction pattern was determined by using Kα doublet of copper.

TABLE 3

| Diffraction angle 2θ (°) (±0.2°) | Lattice spacing d (Å) (±0.1 Å) | Relative intensity I/Io |
|---|---|---|
| 5.6 | 15.8 | 100 |
| 11.2 | 7.89 | 6 |
| 11.8 | 7.49 | 30 |
| 14.8 | 5.98 | 36 |
| 15.3 | 5.79 | 19 |
| 19.4 | 4.57 | 60 |
| 20.2 | 4.39 | 19 |
| 20.5 | 4.33 | 19 |
| 22.7 | 3.91 | 69 |
| 23.4 | 3.80 | 9 |
| 24.3 | 3.66 | 41 |
| 25.6 | 3.48 | 57 |
| 27.2 | 3.28 | 32 |
| 28.0 | 3.18 | 72 |
| 29.1 | 3.07 | 53 |
| 29.8 | 3.00 | 9 |
| 30.8 | 2.91 | 61 |
| 33.8 | 2.65 | 29 |
| 34.2 | 2.62 | 20 |

To confirm the effect of the present invention, Georgia kaolin was incorporated with the above-obtained L type zeolite to prepare a zeolite-containing catalyst. Namely, 10 g of Georgia kaolin having an $SiO_2$ content of 43.8% by weight and an $Al_2O_3$ content of 38.1% by weight was incorporated with 10 g of the L type zeolite, and 200 g of water was further added. The mixture was sufficiently stirred, subjected to evaporation to dryness, pulverized, and calcined in an air current at 540°C for 3 hours. Chlorination reaction of MCB was carried out by using the thus-prepared catalyst.

The reaction was carried out by using an ordinary semi-batchwise reaction vessel made of Pyrex glass and having an inner diameter of 50 mm. In 40 g of MCB was suspended 2 g of the L type zeolite-containing catalyst. Chlorine gas (together with the same amount of nitrogen) was introduced into the vessel at a rate of 26.9 cc/min under agitation by a magnetic stirrer. The reaction temperature was adjusted to 100°C by controlling the temperature of the periphery of the reaction vessel in an oil bath. The composition of the product obtained at the point when 3 hours had passed from the start of introduction of the chlorine gas was analyzed by gas chromatography. The results obtained are shown in Table 4.

Examples 2 through 5

In the same manner as described in Example 1, catalysts were prepared by inoculating Kibushi Kaolin (having an $SiO_2$ content of 44.5% by weight and an $Al_2O_3$ content of 32.7% by weight), acid clay (supplied by Mizusawa Kagaku and having an $SiO_2$ content of 73.8% by weight and an $Al_2O_3$ content of 13.2% by weight), silica-alumina (supplied by Shokubai Kasei and having an $SiO_2$ content of 71.3% by weight and an $Al_2O_3$ content of 28.6% by weight), and sepiolite (supplied by Showa Kogyo and having an $SiO_2$ content of 56.5% by weight and an $Al_2O_3$ content of 2.0% by weight) with the L type zeolite synthesized in Example 1.

By using these catalysts, chlorination of MCB was carried out in the same manner as described in Example 1. The results obtained are shown in Table 4.

Example 6

A catalyst was prepared in the same manner as described in Example 1 except that 20 g of Georgia kaolin was incorporated with the L type zeolite synthesized in Example 1. By using this catalyst, chlorination reaction of MCB was carried out in the same manner as described in Example 1. The results obtained are shown in Table 4.

Example 7

A catalyst was prepared in the same manner as described in Example 1 except that 1 g of sepiolite used in Example 5 was incorporated, instead of 10 g of Georgia kaolin, with the L type zeolite synthesized in Example 1. By using this catalyst, chlorination of MCB was carried out in the same manner as described in Example 1. The results obtained are shown in Table 4.

Comparative Example 1

A catalyst was prepared in the same manner as described in Example 1 except that 5 g of alumina (supplied by Sumitomo Chem. Co.) was incorporated, instead of 10 g of Georgia Kaolin, with the L type zeolite synthesized in Example 1. By using this catalyst, chlorination of MCB was carried out in the same manner as described in Example 1. The results obtained are shown in Table 4.

Comparative Example 2

With 10 g of the L type zeolite synthesized in Example 1 was incorporated 10 g of alumina (supplied by Sumitomo Chem. Co.), and 200 g of water was further added. The mixture was sufficiently stirred, subjected to evaporation to dryness, pulverized, and calcined in an air current at 540°C for 3 hours. By using the thus-prepared catalyst, chlorination of MCB was carried out in the same manner as described in Example 1. The results obtained are shown in Table 4.

Comparative Example 3

A catalyst was prepared in the same manner as described in Comparative Example 2 by incorporating 10 g of alumina (supplied by Mizusawa Kagaku) with the L type zeolite synthesized in Example 1. By using the thus-prepared catalyst, chlorination of MCB was carried out in the same manner as described in Comparative Example 2. The results obtained are shown in Table 4.

TABLE 4

| | Binder | Content of aluminum other than zeolite component (% by weight as $Al_2O_3$) | Conversion (%) of MCB | Selectivities (mole %) to respective products | | | |
|---|---|---|---|---|---|---|---|
| | | | | PDCB | ODCB | MDCB[1)] | Others[2)] |
| Example 1 | Georgia kaolin | 19.1 | 57.1 | 88.2 | 10.9 | 0.8 | 0.1 |
| Example 2 | Kibushi kaolin | 16.4 | 52.5 | 88.5 | 10.6 | 0.8 | 0.1 |
| Example 3 | Acid clay | 6.6 | 52.9 | 87.9 | 10.9 | 1.0 | 0.2 |
| Example 4 | Silica-alumina | 14.3 | 53.6 | 87.0 | 12.1 | 0.8 | 0.1 |
| Example 5 | Sepiolite | 1.0 | 51.1 | 87.6 | 11.5 | 0.8 | 0.1 |
| .Example 6 | Georgia kaolin | 25.4 | 50.3 | 88.3 | 10.6 | 0.8 | 0.2 |
| Example 7 | Sepiolite | 0.18 | 53.1 | 87.6 | 11.4 | 0.9 | 0.1 |
| Comparative Example 1 | Alumina | 31.8 | 52.8 | 82.0 | 16.6 | 1.1 | 0.3 |
| Comparative Example 2 | Alumina | 47.8 | 51.8 | 73.0 | 25.0 | 1.5 | 0.5 |
| Comparative Example 3 | Alumina | 49.0 | 52.8 | 72.5 | 25.4 | 1.6 | 0.5 |

[1] MDCB: metadichlorobenzene
[2)] Others: trichlorobenzenes

Example 8

In 970 ml of 2N aqueous ammonia was suspended 30 g of the L type zeolite synthesized in Example 1, and an ion exchange treatment was carried out at 90°C for 5 hours with stirring. The zeolite was recovered by filtration, washed with water, and then dried at 120°C for 16 hours. The product had the following molar oxide composition (dry base):

$$0.24K_2O \cdot Al_2O_3 \cdot 5.99SiO_2$$

Namely, there was obtained an L type zeolite in which about 76% of exchange sites were occupied by proton. With 10 g of this L type zeolite was incorporated 10 g of Georgia kaolin. Chlorination of MCB was carried out in the same manner as described in Example 1. The conversion of MCB was 54.6% and the selectivity to PDCB was 82.9%.

Example 9

Chlorination of MBB was carried out by using the catalyst prepared in Example 1, according to the same procedures as described in Example 1, except that MBB was used instead of MCB. The conversion of MBB was 64.9%, and the obtained bromochlorobenzene comprised 84.9% of the para-isomer, 13.2% of the ortho-isomer, and 1.9% of the meta-isomer.

Example 10

By using the catalyst prepared in Example 1, chlorination of toluene was carried out in the same manner as described in Example 1 except that 30 g of toluene was used instead of 40 g of MCB. The results obtained after the lapse of 3 hours from the start of the reaction are shown in Table 5.

Comparative Example 4

By using the catalyst prepared in Comparative Example 2, chlorination of toluene was carried out in the same manner as described in Example 9. The results obtained are shown in Table 5.

TABLE 5

| | Binder | Content of aluminum other than zeolite component (% by weight as $Al_2O_3$) | Conversion (%) of toluene | Selectivities (mole %) to respective products | | | |
|---|---|---|---|---|---|---|---|
| | | | | PCT | OCT | MCT | Others |
| Example 9 | Georgia kaolin | 19.1 | 52.3 | 59.7 | 34.6 | 2.1 | 3.6 |
| Comparative Example 4 | Alumina | 47.8 | 55.4 | 44.7 | 46.2 | 3.6 | 5.5 |

Note
PCT: parachlorotoluene
OCT: orthochlorotoluene
MCT: metachlorotoluene
Others: dichlorotoluenes

Example 11

According to the teaching of EP—A 106643, zeolite OE, which is an offretite-erionite type zeolite, was synthesized. More specifically, 23.6 g of solid sodium hydroxide (NaOH=93% by weight), 26.8 g of solid potassium hydroxide (KOH=85% by weight), and 51.4 g of an aqueous solution of sodium aluminate ($Na_2O$=19.2% by weight, $Al_2O_3$=20.66% by weight) were added to 701.2 g of pure water to form an homogeneous solution. Then, 142.2 g of amorphous solid silica (white carbon supplied by Nippon Silica Kogyo) was added to the solution with stirring to obtain a reaction mixture having the following molar oxide composition:

$$3.9Na_2O \cdot 1.83K_2O \cdot Al_2O_3 \cdot 18.8SiO_2 \cdot 382H_2O$$

This mixture was characterized by the following molar ratios:

$$SiO_2/Al_2O_3=18.8$$
$$OH/SiO_2=0.61$$
$$K/(K+Na)=0.32$$
$$H_2O/SiO_2=20.3$$

The mixture was charged in an autoclave and then heated at 150°C for 40 hours with stirring at 250 rpm. The obtained slurry was subjected to solid-liquid separation, and the recovered solid was sufficiently washed with water and dried at 150°C for 5 hours to obtain a powder having the following composition (dry base):

$$0.21Na_2O \cdot 0.76K_2O \cdot Al_2O_3 \cdot 7.4SiO_2$$

The powder X-ray diffraction pattern of the sample, which is shown in Table 6, was substantially in accord with the pattern shown in Table 2, and it was confirmed that the product was an offretite-erionite type zeolite, that is, zeolite OE.

This zeolite OE was ion-exchanged with K. Namely, 35 g of this zeolite OE was added to 250 g of an aqueous solution of potassium chloride (1 mole/l), and the ion exchange treatment was carried out at 90°C for 5 hours. The slurry was then filtered, and the recovered solid was sufficiently washed with water and dried at 120°C for 16 hours. The powder X-ray diffraction pattern of this K-ion-exchanged zeolite OE was not substantially different from that shown in Table 6, and the zeolite had the following composition (dry base):

$$0.01Na_2O \cdot 0.98K_2O \cdot Al_2O_3 \cdot 7.1SiO_2$$

TABLE 6

| Diffraction angle 2θ (°) (±0.2°) | Lattice spacing d (Å) (±0.1 Å) | Relative intensity I/Io |
|---|---|---|
| 7.7 | 11.5 | 100 |
| 9.5 | 9.3 | 8 |
| 11.6 | 7.6 | 27 |
| 13.2 | 6.7 | 85 |
| 13.9 | 6.4 | 17 |
| 15.2 | 5.8 | 13 |
| 16.4 | 5.4 | 7 |
| 19.3 | 4.6 | 15 |
| 20.3 | 4.4 | 73 |
| 21.2 | 4.2 | 9 |
| 23.1 | 3.8 | 33 |
| 23.5 | 3.8 | 140 |
| 24.7 | 3.6 | 87 |
| 26.0 | 3.4 | 7 |
| 26.8 | 3.3 | 33 |
| 28.0 | 3.2 | 26 |
| 28.2 | 3.2 | 57 |
| 30.4 | 2.9 | 14 |
| 31.1 | 2.9 | 83 |
| 31.3 | 2.9 | 117 |
| 33.2 | 2.7 | 27 |
| 36.0 | 2.5 | 28 |

With 10 g of the K-ion-exchanged zeolite OE was incorporated 10 g of Georgia kaolin as the binder, and 200 g of water was further added. The mixture was sufficiently stirred, subjected to evaporation to dryness,

pulverized, and compression-molded to 5 mm (diameter)×5 mm (length). The molded catalyst was calcined in an air current at 540°C for 3 hours.

Chlorination of MCB was carried out as follows. An ordinary fixed bed type reaction apparatus was used. 5 g of the catalyst was packed in a Pyrex reaction tube having a diameter of 20 mm and a length of 500 mm. A starting material mixture comprising MCB, $Cl_2$, and $N_2$ at a ratio of 18/9/73 was supplied at a contact time of 23 g-cat · hr/mole, and the reaction was conducted at 200°C. After the reaction became stationary, the product was sampled and analyzed by gas chromatography. The results obtained are shown in Table 7.

## Examples 12 and 13

In the same manner as described in Example 11, catalysts were prepared by incorporating acid clay (supplied by Mizusawa Kagaku) or silica-alumina (supplied by Shokubai Kasei) to the zeolite OE synthesized in Example 12. A reaction tube was packed with 5 g of the catalyst, and chlorination of MCB was carried out under the same conditions as described in Example 12. The results obtained are shown in Table 7.

## Comparative Example 5

A catalyst was prepared in the same manner as described in Example 11 by incorporating alumina (supplied by Sumitomo Chem. Co.) to the zeolite OE synthesized in Example 11. A reaction tube was packed with 5 g of this catalyst, and chlorination of MCB was carried out under the same conditions as described in Example 10. The results obtained are shown in Table 7.

TABLE 7

| | Binder | Content of aluminum other than zeolite component (% by weight as $Al_2O_3$) | Conversion (%) of MCB | Selectivities (mole %) to respective products | | | |
|---|---|---|---|---|---|---|---|
| | | | | PDCB | ODCB | MDCB | Others |
| Example 11 | Georgina kaolin | 19.1 | 56.3 | 88.9 | 9.0 | 1.2 | 0.9 |
| Example 12 | Acid clay | 6.6 | 56.7 | 86.8 | 10.6 | 1.2 | 1.4 |
| Example 13 | Silica-alumina | 14.3 | 50.3 | 82.2 | 14.4 | 1.6 | 1.8 |
| Comparative Example 5 | Alumina | 47.8 | 45.0 | 67.1 | 26.0 | 2.2 | 4.7 |

**Claims**

1. A process for the preparation of halogenated benzene derivatives, which comprises halogenating benzene or a benzene derivative in the presence of a zeolite catalyst characterised in that the catalyst contains an aluminum compound other than the zeolite component in the catalyst in an amount of 0.01 to 30% by weight as $Al_2O_3$ based on the weight of the catalyst.

2. A process according to claim 1, wherein said zeolite catalyst comprises an L type zeolite having a composition represented by the following oxide molar ratio formula:

$$xM_{2/n}O \cdot Al_2O_3 \cdot ySiO_2 \cdot zH_2O$$

wherein M is a cation, n is a valency of the cation M, x is a number of 0.7 to 1.3, y is a number of 4 to 8, and z is a number varying depending upon the extent of the hydration of the zeolite.

3. A process according to claim 1, wherein said zeolite catalyst comprises an offretite-erionite type zeolite having a composition represented by the following oxide molar ratio formula:

$$xM_{2/n}O \cdot Al_2O_3 \cdot ySiO_2 \cdot zH_2O$$

wherein M is a cation, n is a valency of the cation M, x is a number of 0.7 to 1.3, y is a number of 5 to 10, and z is a number varying depending upon the extent of the hydration of the zeolite.

4. A process according to claim 3, wherein at least 70% of the cation M is at least one metal ion selected from the group consisting of potassium, rubidium, cesium, calcium, strontium, and barium.

5. A process according to any one of the preceding claims, wherein said zeolite catalyst is a molded product.

6. A process according to claim 5 wherein the catalyst is a moulded product of a mixture of zeolite powder and 10 to 70% by weight of a binder selected from the group consisting of synthetic inorganic oxides and natural or refined clays.

7. A process according to claim 5 or claim 6 wherein said moulded product is calcined at a temperature of 300°C to 700°C.

8. A process according to any one of the preceding claims, wherein the benzene derivative is selected from the group consisting of monochlorobenzene, monofluorobenzene, monobromobenzene, monoiodobenzene, toluene, and ethylbenzene.

9. A process according to any one of the preceding claims, wherein said halogenation of the benzene or benzene derivative is carried out in the liquid phase at a temperature of 0°C to 400°C by using an elementary halogen as a halogenating agent.

10. A process according to any one of claims 1 to 8, wherein said halogenation of the benzene or benzene derivative is carried out in the gas phase at a temperature of 100°C to 400°C by supplying a gaseous feed comprising (a) the benzene or benzene derivative, (b) 0.01 to 5.0 moles of an elementary halogen as a halogenating agent per mole of the benzene or benzene derivative and (c) an inert diluent gas at a rate such that the contact time is in the range of 5 to 500 g-cat · hr/mole.

**Patentansprüche**

1. Verfahren zur Herstellung von halogenierten Benzolderivaten, das darin besteht, daß Benzol oder ein Benzolderivat in Anwesenheit eines Zeolith-Katalysators halogeniert wird, dadurch gekennzeichnet, daß der Katalysator eine von dem Zeolith-Bestandteil des Katalysators unterschiedliche Aluminiumverbindung in einer Menge von 0,01 bis 30 Gew.-% als $Al_2O_3$ auf der Basis des Gewichts des Katalysators aufweist.

2. Verfahren gemäß Anspruch 1, worin der Zeolith-Katalysator einen Zeolith vom L-Typ mit einer Zusammensetzung ist, die durch die folgende Formel auf Basis des molaren Verhältnisses der Oxyde zueinander wiedergegeben ist:

$$xM_{2/n}O \cdot Al_2O_3 \cdot ySiO_2 \cdot zH_2O$$

worin M ein Kation ist, n eine Wertigkeit des Kations M, x eine Zahl von 0,7 bis 1,3 ist, y eine Zahl von 4 bis 8 ist und z eine variable Zahl ist, deren Größe von dem Grad der Hydratisierung des Zeoliths abhängt.

3. Verfahren gemäß Anspruch 1, worin der Zeolith-Katalysator ein Zeolith vom Typ Offretit-Erionit mit einer Zusammensetzung ist, die der folgenden Formel auf der Basis des molaren Verhältnisses der Oxyde zueinander entspricht:

$$xM_{2/n}O \cdot Al_2O_3 \cdot ySiO_2 \cdot zH_2O$$

worin M ein Kation ist, n eines Wertigkeit des Kations M, x eine Zahl von 0,7 bis 1,3, y eine Zahl von 5 bis 10 und z eine variable Zahl ist, deren Größe von dem Grad der Hydratisierung des Zeoliths abhängt.

4. Verfahren gemäß Anspruch 3, worin mindestens 70% des Kations M mindestens ein Metallisches Ion ausgewählt aus der Gruppe bestehend aus Kalium, Rubidium, Cäsium, Kalzium, Strontium und Barium ist.

5. Verfahren gemäß iregendeinem der vorstehenden Ansprüche, worin der Zeolith-Katalysator ein geschmolzenes Produkt ist.

6. Verfahren gemäß Anspruch 5, worin der Katalysator ein geschmolzenes Produkt aus einem Gemisch aus Zeolith-Pulver und 10 bis 70 Gew.-% eines Bindemittels ist, das ausgewählt ist aus der Gruppe bestehend aus synthetischen anorganischen Oxyden und natürlichen oder raffinierten Tonen.

7. Verfahren gemäß Anspruch 5 oder 6, worin das geschmolzene Produkt bei einer Temperatur von 300 bis 700°C kalziniert ist.

8. Verfahren gemäß iregendeinem der vorstehenden Ansprüche, worin das Benzolderivat ausgewählt ist aus der Gruppe bestehend aus Monochlorbenzol, Monofluorbenzol, Monobrombenzol, Monolotbenzol, Toluol und Ethylbenzol.

9. Verfahren gemäß irgendeinem der vorstehenden Ansprüche, worin die Halogenierung des Benzols oder Benzolderivats in flüssiger Phase bei einer Temperatur von 0 bis 400°C unter Verwendung eines elementaren Halogens als Halogenierungsmittel durchgeführt wird.

10. Verfahren nach einem der Ansprüche 1 bis 8, worin die Halogenierung des Benzols oder Benzolderivats in der Gasphase bei einer Temperatur von 100 bis 400°C und Einführung eines gasförmigen Ausgangsprodukts durchgeführt wird, darin bestehend, daß (a) das Benzol oder Benzolderivat, (b) 0,01 bis 5,0 Mol eines elementaren Halogens als Halogenierungsmittel pro Mol Benzol oder Benzolderivat und (c) ein inertes Vedünnungsgas in einem solchen Verhältnis eingesetzt wird, daß die Kontaktzeit im Bereich von 5 bis 500 g×hr/Mol Katalysator beträgt.

**Revendications**

1. Procédé pour la préparation de dérivés halogénés du benzène, qui comprend l'halogénation du benzène ou d'un dérivé du benzène en présence d'un catalyseur contenant une zéolite, caractérisé en ce que le catalyseur contient un composé d'aluminium autre que le composant de la zéolite dans le catalyseur, en une quantité de 0,01 à 30% en poids sous forme de $Al_2O_3$ sur la base du poids du catalyseur.

2. Procédé selon la revendication 1, selon lequel le catalyseur contenant de la zéolite comprend une zéolite de type L ayant une composition représentée par la formule suivante des rapports molaires des oxydes:

$$xM_{2/n}O \cdot Al_2O_3 \cdot ySiO_2 \cdot zH_2O$$

dans laquelle M est un cation, n est une valence du cation M, x est un nombre de 0,7 à 1,3, y est un nombre de 4 à 8, et z est un nombre variable dépendant de l'importance de l'hydratation de la zéolite.

3. Procédé selon la revendication 1, selon lequel le catalyseur contenant la zéolite comprend une zéolite de type offretite-érionite ayant une composition représentée par la formule suivante des rapports molaires des oxydes:

$$xM_{2/n}O \cdot Al_2O_3 \cdot ySiO_2 \cdot zH_2O$$

dans laquelle M est un cation, n est une valence du cation M, x est un nombre de 0,7 à 1,3, y est un nombre de 5 à 10, et z est un nombre variable dépendant de l'importance de l'hydratation de la zéolite.

4. Procédé selon la revendication 3, selon lequel au moins 70% du cation M est au moins un ion de métal choisi dans le groupe composé du potassium, du rubidium, du césium, du calcium, du strontium et du baryum.

5. Procédé selon l'une quelconque des revendications précédentes, selon lequel le catalyseur contenant de la zéolite est un produit moulé.

6. Procédé selon la revendication 5, selon lequel le catalyseur est un produit moulé d'un mélange de poudre de zéolite et de 10 à 70% en poids d'un liant choisi dans le groupe composé des oxydes inorganiques synthétiques et des argiles naturelles ou raffinées.

7. Procédé selon la revendication 5 ou la revendication 6, selon lequel le produit moulé est calciné à une température de 300°C à 700°C.

8. Procédé selon l'une quelconque des revendications précédentes, selon lequel le dérivé du benzène est choisi dans le groupe composé de: monochlorobenzène, monofluorobenzène, monobromobenzène, monoiodobenzène, toluène et éthylbenzène.

9. Procédé selon l'une quelconque des revendications précédentes, selon lequel l'halogénation du benzène ou du dérivé du benzène est effectuée en phase liquide à une température de 0°C à 400°C par l'emploi d'un halogène élémentaire servant d'agent d'halogénation.

10. Procédé selon l'une quelconque des revendications 1 à 8, selon lequel l'halogénation du benzène ou du dérivé du benzène est effectuée en phase gazeuse à une température de 100°C à 400°C par la fourniture d'une alimentation gazeuse comprenant (a) le benzène ou le dérivé du benzène, (b) 0,01 à 5 moles d'un halogène élémentaire servant d'agent d'halogénation par mole du benzène ou du dérivé du benzène et (c) un gaz diluant inerte à un débit tel que la durée du contact se trouve dans la gamme de 5 à 500 g-cat · h/mole.